(19) Europäisches Patentamt / European Patent Office / Office européen des brevets

(11) EP 2 802 383 B1

(12) **EUROPÄISCHE PATENTSCHRIFT**

(45) Veröffentlichungstag und Bekanntmachung des Hinweises auf die Patenterteilung:
**03.01.2018 Patentblatt 2018/01**

(21) Anmeldenummer: **12781099.2**

(22) Anmeldetag: **05.11.2012**

(51) Int Cl.:
***A61Q 5/00*** $^{(2006.01)}$ ***A61K 8/73*** $^{(2006.01)}$
***A61Q 5/10*** $^{(2006.01)}$

(86) Internationale Anmeldenummer:
**PCT/EP2012/071819**

(87) Internationale Veröffentlichungsnummer:
**WO 2013/087296 (20.06.2013 Gazette 2013/25)**

# (54) VERWENDUNG VON PROPYLENOXID-MODIFIZIERTER STÄRKE ZUR VERBESSERUNG KÜNSTLICHER FÄRBUNGEN KERATINISCHER FASERN

USE OF PROPYLENE OXIDE-MODIFIED STARCH FOR IMPROVING ARTIFICIAL COLOURING OF KERATIN FIBRES

UTILISATION D'AMIDON MODIFIÉ PAR OXYDE DE PROPYLÈNE POUR AMÉLIORER LA COLORATION DE FIBRES KERATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011088397**

(43) Veröffentlichungstag der Anmeldung:
**19.11.2014 Patentblatt 2014/47**

(73) Patentinhaber: **Henkel AG & Co. KGaA**
**40589 Düsseldorf (DE)**

(72) Erfinder: **SCHWEINSBERG, Matthias**
**22769 Hamburg (DE)**

(56) Entgegenhaltungen:
**EP-A1- 1 568 351**
**EP-A2- 0 761 200**
**WO-A1-2005/034893**
**WO-A1-2005/082321**
**WO-A1-2012/084904**
**WO-A2-2013/023855**
**FR-A1- 2 944 967**

- **DATABASE WPI Week 201067 Thomson Scientific, London, GB; AN 2010-M48909 XP002728050, & JP 2010 215599 A (MANDOM KK) 30. September 2010 (2010-09-30)**
- **SINGH ET AL: "Factors influencing the physico-chemical, morphological, thermal and rheological properties of some chemically modified starches for food applications-A review", FOOD HYDROCOLLOIDS, ELSEVIER BV, NL, Bd. 21, Nr. 1, 1. Januar 2007 (2007-01-01) , Seiten 1-22, XP005664085, ISSN: 0268-005X, DOI: 10.1016/J.FOODHYD.2006.02.006**
- **SAOWAKON WATTANACHANT ET AL: "Effect of crosslinking reagents and hydroxypropylation levels on dual-modified sago starch properties", FOOD CHEMISTRY, Bd. 80, Nr. 4, 1. April 2003 (2003-04-01), Seiten 463-471, XP055132395, ISSN: 0308-8146, DOI: 10.1016/S0308-8146(02)00314-X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 802 383 B1

Printed by Jouve, 75001 PARIS (FR)

## Beschreibung

Die vorliegende Erfindung betrifft kosmetische Mittel zur Färbung keratinischer Fasern, enthaltend in einem kosmetischen Träger mindestens eine farbgebende Verbindung und mindestens eine mittels Propylenoxid modifizierte Stärke, welche ein mittleres Molekulargewicht (Gewichtsmittel) von 50 bis 2500 kDa besitzt und einen Propylenoxidgehalt von 0.1 bis 20, 0 Gew.-% (bezogen auf das Gewicht der mit Propylenoxid modifizierten Stärke) aufweist.

Zur Bereitstellung kosmetischer Mittel zur Färbung, insbesondere für keratinische Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte dierektziehende Farbstoffe enthalten. Hierbei handelt es sich um Farbstoffmoleküle, die direkt auf das Substrat aufziehen und keinen oxidativen Prozeß zur Ausbildung der Farbe benötigen. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Es ist ein stetes Anliegen, die Färbeleistung von Färbemitteln zu verbessern. Aus der EP-A1-1 568 351 ist die Verwendung einer Zusammensetzung umfassend eine vorgelatinierte Amylose enthaltende Stärke zur Haltbarmachung künstlicher Farbe auf Haaren bekannt.

Die WO-A1-98/01109 betrifft Mittel zur Reinigung oder Pflege von Haar, enthaltend eine vorverkleisterte, vernetzte Stärke, ausgewählt aus einer ($C_2$ bis $C_6$)-Hydroxyalkylstärke und einer ($C_2$ bis $C_6$)-Acylstärke.

Die WO 2005/082321 A1 offenbart die Verwendung eines kationischen Stärkederivates zur Stärkung der inneren und/oder äußeren Struktur keratinischer Fasern und/oder zur Erzielung verbesserter Echtheitseigenschaften von gefärbten und/oder biondierten keratinischen Fasern bzw. zur Erhöhung der Echtheiten gefärbter und/oder blondierter keratinischer Fasern, wobei die Stärkederivate Molekulargewichte im Bereich von 100 bis 50.000 kDa aufwweisen sollen.

Allerdings sind die Mittel des Standes der Technik noch immer verbesserungswürdig,

Eine Verbesserung beispielsweise der Farbintensität oder der Färbekraft der eingesetzten Farbstoffe hat z.B. zur Folge, dass teure Farbstoffe wirtschaftlicher eingesetzt werden können. Die erzielten Färbungen sollen weiterhin ein hohes Maß an Farbechtheit z.B. gegen Schweiß, Waschen, Licht oder Reibung aufweisen und müssen insbesondere im Rahmen der Haarpflege kompatibel mit der Anwendung anderer Haarbehandlungsmitteln sein. Eine gleichmäßige Färbung entlang der keratinischen Fasern stellt ebenso eine Anforderung an ein kommerziell erfolgreiches Färbemittel für keratinische Fasern dar. Keratinische Fasern, insbesondere menschliche Haare, sind als gewachsene Naturprodukte ungleichmäßig bezüglich ihrer strukturellen Beschaffenheit entlang der Faser. Das keratinische Material der Faser in den Haarlängen und den Haarspitzen wurde beispielsweise über einen längeren Zeitraum der Umwelteinflüsse ausgesetzt als die Regionen der Faser in der Nähe der Haarwurzel und weisen daher stärkere Veränderungen der ursprünglich gewachsenen Faserstruktur auf. Unterschiede in der Faserstruktur führten oftmals zu einer ungleichmäßigen Farbaufnahme sowie zu einer ungleichmäßigen Abnutzung der Färbung durch Umwelteinflüsse. Die Färbung wird visuell als ungleichmäßig wahrgenommen.

Aufgabe der vorliegende Erfindung war es daher, eine die keratinischen Fasern färbende, kosmetische Zusammensetzung bereitzustellen, die eine verbesserte Färbung bewirkt und die vorgenannten Nachteile nicht aufweist.

Ein erster Gegenstand ist daher ein kosmetisches Mittel zur Färbung keratinischer Fasern, insbesondere menschlicher Haare, enthaltend in einem kosmetischen Träger mindestens eine farbgebende Verbindung und mindestens eine mittels Propylenoxid modifizierte Stärke, welche ein mittleres Molekulargewicht (Gewichtsmittel) von 700 bis 1000 kDa und einen Propylenaxidgehalt von 2,0 bis 12,0 Gew.-% (bezogen auf das Gewicht der mittels Propylenoxid modifizierten Stärke) besitzt, wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird,

wobei die mittels Propylenoxid modifizierte Stärke eine mittels Propylenoxid modifizierte Tapiokastärke oder eine mittels Propylenoxid modifizierte Kartoffelstärke oder eine mittels Propylenoxid modifizierte Maisstärke oder ein Gemisch aus beiden vorgenannten Stärken, ist, und

wobei zusätzlich mindestens ein Oxidationsmittel, insbesondere Wasserstoffperoxyd und/oder mindestens ein Anlagerungsprodukt davon, enthalten ist.

Es stellte sich heraus, dass der Einsatz der besagten, mittels Propylenoxid modifizierten Stärke auf bereits gefärbter keratinischer Faser (insbesondere während des Färbevorganges), die Farbechtheit der Färbung, insbesondere die Waschechtheit der Färbung, verbessert. Durch mehrmaliges Waschen der Faser entsteht entlang der keratinischen Faser vom Ansatz bis in die Spitzen keine ungleichmäßige Färbung durch ungleichmäßiges Auswaschen der Farbe. Die Färbung behält ihre gute Egalisierung.

Unter keratinischen Fasern sind erfindungsgemäß Pelze, Wolle, Federn und insbesondere menschliche Haare zu verstehen.

Stärke ist ein Reservekohlenhydrat, das von vielen Pflanzen in Form von üblicherweise 1 bis 200 $\mu$m großen Stärkekörnern (Granula) in verschiedenen Pflanzenteilen gespeichert wird, z.B. in Knollen oder Wurzeln, Getreidesamen, Früchten sowie im Mark. Erfindungsgemäß ist die mittels Propylenoxid modifizierte Stärke eine mittels Propylenoxid modifizierte Tapioka Stärke oder eine mittels Propylenoxid modifizierte Kartoffelstärke oder eine mittels Propylenoxid modifizierte Maisstärke oder ein Gemisch aus beiden vorgenannten Stärken,.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten zwischen 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die mittels Propylenoxid modifizierte Stärke einen Amylosegehalt von kleiner 25 Gew.-%, insbesondere von kleiner 20 Gew.-%, - jeweils bezogen auf das Gewischt der besagten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear $\alpha$-1,4-glycosidisch verknüpfter d-Glucose, $M_r$ 50000-150000. Die resultierenden Ketten bilden in der Stärke Doppelhelices.

Amylopektin enthält neben den für Amylose beschriebenen $\alpha$-1,4-Verknüpfungen auch in einer Menge von 4 bis 6% $\alpha$-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer mittels Propylenoxid modifizierten Stärke wird erfindungsgemäß ein Reaktionsprodukt einer Stärke mit Propylenoxid verstanden. Ein solches Reaktionsprodukt umfasst mindestens eine Struktureinheit der Formel (I),

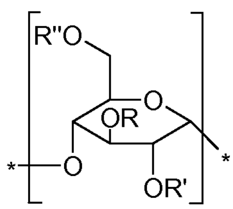

(I),

worin mindestens ein Rest R, R' oder R" für eine Gruppe der Formel

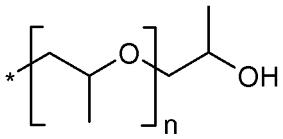

mit n≥0 steht

und maximal 2 der Reste aus R, R', R" für ein Wasserstoffatom steht. Eine mit dem Symbol * gekennzeichnete Bindung in Formeln dieser Anmeldung entspricht einer freien Valenz der entsprechenden Struktureinheit. Die entsprechend mittels Propylenoxid modifizierten Stärken werden beispielsweise durch Umsetzung einer nativen Stärke mit Propylenoxid bereitgestellt. Vor der Modifikation mittels Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung etc.

Es ist erfindungsgemäß bevorzugt, wenn die mittels Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mit Propylenoxid modifiziert.

Im Rahmen einer bevorzugten Ausführungsform ist die mittels Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen mittels Propylenoxid modifizierten Stärken liegen in dem erfindungsgemäßen Mittel in einer Molekulargewichtsverteilung vor. Die Molekulargewichtsverteilung wurde experimentell mittels Gelfiltrationschromatographie gegen Dextran bestimmt. Ein wichtiges Merkmal der Erfindung ist das Gewichtsmittel des mittleren Molekulargewichts der in dem erfindungsgemäßen Mittel enthaltenen mittels Propylenoxid modifizierten Stärken. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus *i* Segmenten (z. B. Monomerbausteinen) der Masse $M_I$ bestehen und in der Probe $N_i$-mal vorkommen. Für das Gewichtsmittel des Molekulargewichts $M_w = \Sigma_{WI} M_i$ gilt damit

$$M_w = [\Sigma(N_i M_i^2)] / [\Sigma(N_i M_i)].$$

Die erfindungsgemäßen Mittel enthalten mindestens eine mittels Propylenoxid modifizierte Stärke, die ein mittleres Molekulargewicht (Gewichtsmittel) von 700 bis 1000 kDa, aufweist.

Es ist erfindungsgemäß besonders bevorzugt, wenn die mittels Propylenoxid modifizierte Stärke unvernetzt ist. Eine Vernetzung der mittels Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln wie beispielsweise mit Epichlorhydrin durchrühren. (Mittels Propylenoxid modifizierte) Stärken, die eine solche Vernetzung nicht aufweisen, sind unvernetzt.

Zur Erzielung eines niedrigeren Molekulargewichts z.B. von 700-900 kDa, werden besagte Stärken einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, extruder, Stator/Rotor-Maschinen und/oder Rührwerke.

Die oxidative Spaltung mittels Wasserstoffperoxid eignet sich bevorzugt. Zu diesem Zweck wird beispielsweise die mittels Propylenoxid modifizierte Stärke in Wasser gegeben, auf 50 bis 70 °C erhitzt, Wasserstoffperoxid zugefügt

und bei 70 bis 85°C für 2 bis 5 Stunden gerührt.

Der Gehalt an Propylenoxid der Stärke wirkt sich auf die Feinabstimmung der Eigenschaften der erhaltenen Färbung und der Stabilität der kosmetischen Mittel aus. Erfindungsgemäß beträgt der Propylenoxidgehalt 2 bis 12 Gew.-%. Es zeigte sich, dass die Parameter weiter optimiert werden, wenn die besagte mittels Propylenoxid modifizierte Stärke - bezogen auf das Gewicht der modifizierten Stärke einen Propylenoxidgehalt von 3,0 bis 10,0 Gew.-%, ganz besonders bevorzugt einen Propylenoxidgehalt von 4,0 bis 6,0 Gew.-%, aufweiset. Der Propylenoxidgehalt lässt sich beispielsweise nach Durchführung einer Hodges-Spaltung mit der Methode gemäß DIN EN 13268 bestimmen.

Ferner hat es sich erwiesen, dass solche kosmetischen Mittel sich im Sinne der Erfindung hervorragend eignen, in denen die besagte mittels Propylenoxid modifizierte Starke in einer 43 Gew.-%-igen Lösung in Wasser (i.e. einer43 Gew.-%-igen wässrigen Lösung) eine Viskosität im Bereich von 150 bis 1500000 mPa·s (Brookfield Viskosimeter, Spindel #7 bei 20°C und 20 U/min) aufweist. Hervorragend geeignete Propylenoxid modifizierte Polysaccharide weisen Viskositäten von 3000 bis 200000 mPa·s, insbesondere von 10000 bis 100000 mPa·s, ganz besonders bevorzugt von 40000 bis 70000 mPa·s, (jeweils gemessen unter den zuvor genannten Bedingungen) auf.

Bevorzugte kosmetische Trager sind wasserhaltige kosmetische Träger, alkoholische kosmetische Träger oder wässrig-alkoholische kosmetische Träger. Zum Zwecke der Färbung keratmischer Fasern sind solche Träger beispielsweise Lotionen, Wasser-in-Öl-Emulsionen, Öl-n-Wasser-Emulsionen, Cremes, Gele, Schäume oder andere Zubereitungen, die für die Anwendung auf dem Haar geeignet sind.

Unter wässrig-alkoholischen Trägern sind im Sinne der vorliegenden Erfindung wässrige Zusammensetzungen enthaltend 3 bis 70 Gew,-% eines organischen Lösemittels, insbesondere eines $C_1$-$C_7$-Alkohols (bevorzugt Ethanol, Isopropanol, Glyzerin, Benzylalkohol, Ethyldiglykol, 1,2-Propylenglykol oder 1,3-Propylenglykol, Methoxybutanol,) zu verstehen. Bevorzugt sind dabei alle wasserlöslichen organischen Lösemittel.

Erfindungsgemaße Mittel enthalten mindestens eine farbgebende Verbindung, die ausgewählt ist aus mindestens einem Oxidationsfarbstoffvorprodukt vom Typ der Entwicklerkomponenten und gegebenenfalls zusätzlich mindestens einer Kupplerkomponente

Es ist erfindungsgemäß bevorzugt, als Entwicklerkomponente ein p-Phenylendiaminderivat oder eines seiner physiologisch verträglichen Salze einzusetzen. Besonders bevorzugt sind p-Phenylendiaminderivate der Formel (E1)

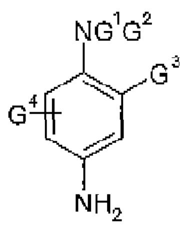

(E1)

wobei

- $G^1$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen 4'-Aminaphenylrest oder einen ($C_1$ bis $C_4$)-Alkylrest, der mit einer stickstoffhaltigen Gruppe, einem Phenyl- oder einem 4'-Aminophenylrest substituiert ist;
- $G^2$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest oder einen ($C_1$ bis $C_4$)-Alkylrest, der mit einer stickstoffhaltigen Gruppe substituiert ist;
- $G^3$ steht für ein Wasserstoffatom, ein Halogenatom, wie ein Chlor-, Brom-, lod- oder Fluoratom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Hydroxyalkoxyrest, einen ($C_1$ bis $C_4$)-Alkoxy-($C_1$ bis $C_4$)-alkylrest, einen ($C_1$ bis $C_4$)-Acetylaminoalkoxyrest, einen Mesylamino-($C_1$ bis $C_4$)-alkoxyrest oder einen ($C_1$ bis $C_4$)-Carbamoylaminoalkoxyrest;
- $G^4$ steht für ein Wasserstoffatom, ein Halogenatom, einen ($C_1$ bis $C_4$)-Alkylrest oder einen ($C_1$ bis $C_4$)-Alkoxy-($C_1$ bis $C_4$)-alkylrest, oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-((3-hydroxyethyl)-p-phenylendiamin, 4-N,N-Bis-((3-hydroxyethyl)-amino-2-methylanilin, 4-N,N-Bis-(β-hydroxyethyl)-amino-2-chloranilin, 2-((3-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-((3-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-((3,y-Dihydroxypropyl)-p-phenylendiamin, N-(4'-Aminophenyl)-p-phenylendiamin, N-Phenyl-p-phenylendiamin, 2-((3-Hydroxyethyloxy)-p-phenylendiamin, 2-Methoxymethyl-p-phenylendiamin, 2-((3-Acetylaminoethyloxy)-p-phenylendiamin, N-((3-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-((3-Hydroxyethyl)-p-phenylendiamin, 2-(α,β-Dihydroxyethyl)-p-phenylendiamin, N,N-Bis-((β-hydroxyethyl)-p-phenylendiamin, 2-Methoxymethyl-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

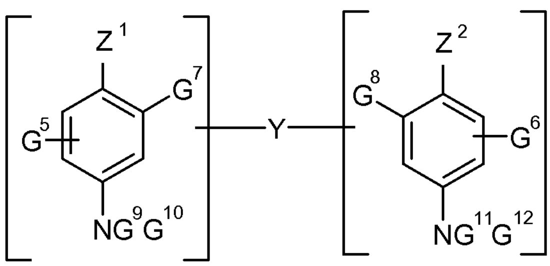

(E2)

wobei:

- $Z^1$ und $Z^2$ stehen unabhängig voneinander für einen Hydroxyl- oder $NH_2$-Rest, der gegebenenfalls durch einen ($C_1$ bis $C_4$)-Alkylrest, durch einen ($C_1$ bis $C_4$)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder ($C_1$ bis $C_8$)-Alkoxyreste substituiert sein kann, oder eine direkte Bindung,
- $G^5$ und $G^6$ stehen unabhängig voneinander für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen ($C_1$ bis $C_4$)-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-((3-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter

N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin ist es erfindungsgemäß bevorzugt, als Entwicklerkomponente mindestens ein p-Aminophenolderivat oder eines seiner physiologisch verträglichen Salze einzusetzen. Besonders bevorzugt sind p-Aminophenolderivate der Formel (E3)

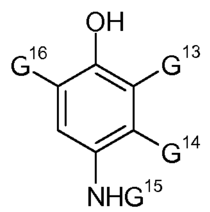

(E3)

wobei:

- $G^{13}$ steht für ein Wasserstoffatom, ein Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest, einen Hydroxy-($C_1$ bis $C_4$)-alkylaminorest, einen ($C_1$ bis $C_4$)-Hydroxyalkoxyrest, einen ($C_1$ bis $C_4$)-Hydroxyalkyl-($C_1$ bis $C_4$)-aminoalkylrest oder einen (Di-[($C_1$ bis $C_4$)-alkyl]amino)-($C_1$ bis $C_4$)-alkylrest, und
- $G^{14}$ steht für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest oder einen ($C_1$ bis $C_4$)-Cyanoalkylrest,
- $G^{15}$ steht für Wasserstoff, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin wird die Entwicklerkomponente bevorzugt ausgewählt aus heterocyclischen Entwicklerkomponenten, wie beispielsweise aus Pyrimidinderivaten, Pyrazolderivaten, bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen,

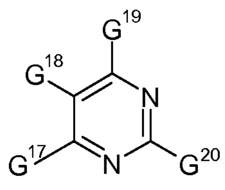

(E4)

worin

- $G^{17}$, $G^{18}$ und $G^{19}$ unabhängig voneinander für ein Wasserstoffatom, eine Hydroxygruppe, eine ($C_1$ bis $C_4$)-Alkoxygruppe oder eine Aminogruppe steht und

- $G^{20}$ für eine Hydroxygruppe oder eine Gruppe $-NG^{21}G^{22}$ steht, worin $G^{21}$ und $G^{22}$ unabhängig voneinander stehen für ein Wasserstoffatom, eine ($C_1$ bis $C_4$)-Alkylgruppe, eine ($C_1$ bis $C_4$)-Monohydroxyalkylgruppe,

mit der Maßgabe, dass maximal zwei der Gruppen aus $G^{17}$, $G^{18}$, $G^{19}$ und $G^{20}$ eine Hydroxygruppe bedeuten und höchstens zwei der Reste $G^{17}$, $G^{18}$ und $G^{19}$ für ein Wasserstoffatom stehen. Dabei ist es wiederum bevorzugt, wenn gemäß Formel (E4) mindestens zwei Gruppen aus $G^{17}$, $G^{18}$, $G^{19}$ und $G^{20}$ für eine Gruppe $-NG^{21}G^{22}$ stehen und höchstens zwei Gruppen aus $G^{17}$, $G^{18}$, $G^{19}$ und $G^{20}$ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5),

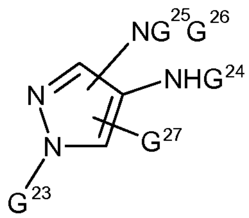

(E5)

worin

- $G^{23}$, $G^{24}$, $G^{25}$ stehen unabhängig voneinander für ein Wasserstoffatom, eine ($C_1$ bis $C_4$)-Alkylgruppe, eine ($C_1$ bis $C_4$)-Monohydroxyalkylgruppe, eine ($C_2$ bis $C_4$)-Polyhydroxyalkylgruppe, eine gegebenenfalls substituierte Arylgruppe oder eine gegebenenfalls substituierte Aryl-($C_1$ bis $C_4$)-alkylgruppe, mit der Maßgabe dass, wenn $G^{25}$ für ein Wasserstoffatom steht, $G^{27}$ neben den vorgenannten Gruppen zusätzlich für eine Gruppe - $NH_2$ stehen kann,
- $G^{26}$ steht für ein Wasserstoffatom, eine ($C_1$ bis $C_4$)-Alkylgruppe, eine ($C_1$ bis $C_4$)-Monohydroxyalkylgruppe oder eine ($C_2$ bis $C_4$)-Polyhydroxyalkylgruppe und
- $G^{27}$ steht für ein Wasserstoffatom, eine gegebenenfalls substituierte Arylgruppe, eine ($C_1$ bis $C_4$)-Alkylgruppe oder eine ($C_1$ bis $C_4$)-Monohydroxyalkylgruppe, insbesondere für ein Wasserstoffatom oder eine Methylgruppe.

Bevorzugt bindet in Formel (E5) der Rest $-NG^{25}G^{26}$ an die 5 Position und der Rest $G^{27}$ an die 3 Position des Pyrazolzyklus.

Besonders bevorzugte Pyrazol-Derivate sind insbesondere die Verbindungen, die ausgewählt werden aus 4,5-Diamino-1-methylpyrazol, 4,5-Diamino-1-((3-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methyl-pyrazol, 4,5-Diamino-3-hydroxymethyl-1-isopropylpyrazol, 4,5-Diamino-3-methyl-1-isopropyl-pyrazol, 4-Amino-5-(β-aminoethyl)amino-1,3-dimethylpyrazol, sowie deren physiologisch verträglichen Salze.

Im folgenden werden Beispiele für die als Substituenten der Verbindungen der Formeln (E1) bis (E5) genannten Reste aufgezählt: Beispiele für ($C_1$ bis $C_4$)-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $- C(CH_3)_3$. Erfindungsgemäße Beispiele für ($C_1$ bis $C_4$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $- OC(CH_3)_3$, insbesondere eine Methoxy- oder eine Ethoxygruppe.

Weiterhin können als bevorzugte Beispiele für eine ($C_1$ bis $C_4$)-Monohydroxyalkylgruppe $-CH_2OH$, $-CH_2CH_2OH$, $-CH_2CH_2CH_2OH$, $-CHCH(OH)CH_3$, $-CH_2CH_2CH_2CH_2OH$, wobei die Gruppe $-CH_2CH_2OH$ bevorzugt ist. Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl- oder Br-Atome, Cl-Atome sind ganz besonders bevorzugte Beispiele. Beispiele für stickstoffhaltige Gruppen sind insbesondere $-NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und $-NH_3^+$. Beispiele für ($C_1$ bis $C_4$)-Monoalkylaminogruppen sind $-NHCH_3$, $-NHCH_2CH_3$, $-NHCH_2CH_2CH_3$, $-NHCH(CH_3)_2$.

Beispiele für ($C_1$ bis $C_4$)-Dialkylaminogruppe sind $-N(CH_3)_2$, $-N(CH_2CH_3)_2$. Beispiele für ($C_1$ bis $C_4$)-Trialkylammoniumgruppen sind $-N^+(CH_3)_3$, $-N^+(CH_3)_2(CH_2CH_3)$, $-N^+(CH_3)(CH_2CH_3)_2$.

Beispiele für ($C_1$ bis $C_4$)-Hydroxyalkylaminoreste sind $-NH-CH_2CH_2OH$, $-NH-CH_2CH_2OH$, -NH-

$CH_2CH_2CH_2OH$, $-NH-CH_2CH_2CH_2OH$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2-O-CH_3$, $-CH_2CH_2-O-CH_2CH_3$, $-CH_2CH_2CH_2-O-CH_2CH_3$, $-CH_2CH_2-O-CH(CH_3)$, $-CH_2CH_2CH_2-O-CH(CH_3)$.

Beispiele für Hydroxy-($C_1$ bis $C_4$)-alkoxyreste sind $-O-CH_2OH$, $-O-CH_2CH_2OH$, $-O-CH_2CH_2CH_2OH$, $-O-CHCH(OH)CH_3$, $-O-CH_2CH_2CH_2CH_2OH$. Beispiele für ($C_1$ bis $C_4$)-Acetylaminoalkoxyreste sind $-O-CH_2NHC(O)CH_3$, $-O-CH_2CH_2NHC(O)CH_3$, $-O-CH_2CH_2CH_2NHC(O)CH_3$, $-O-CH_2CH(NHC(O)CH_3)CH_3$, $-O-CH_2CH_2CH_2CH_2NHC(O)CH_3$. Beispiele für ($C_1$ bis $C_4$)-Carbamoylaminoalkoxyreste sind $-O-CH_2CH_2-NH-C(O)-NH_2$, $-O-CH_2CH_2CH_2-NH-C(O)-NH_2$, $-O-CH_2CH_2CH_2CH_2-NH-C(O)-NH_2$. Beispiele für ($C_1$ bis $C_4$)-Aminoalkylreste sind $-CH_2NH_2$, $-CH_2CH_2NH_2$, $-CH_2CH_2CH_2NH_2$, $-CH_2CH(NH_2)CH_3$, $-CH_2CH_2CH_2CH_2NH_2$.

Beispiele für ($C_1$ bis $C_4$)-Cyanoalkylreste sind $-CH_2CN$, $-CH_2CH_2CN$, $-CH_2CH_2CH_2CN$. Beispiele für ($C_1$ bis $C_4$)-Hydroxyalkylamino-($C_1$ bis $C_4$)-alkylreste sind $-CH_2CH_2NH-CH_2CH_2OH$, $-CH_2CH_2CH_2NH-CH_2CH_2OH$, $-CH_2CH_2NH-CH_2CH_2CH_2OH$, $-CH_2CH_2CH_2NH-CH_2CH_2CH_2OH$.

Beispiele für Di[($C_1$ bis $C_4$)-Hydroxyalkyl]amino-($C_1$ bis $C_4$)-alkylreste sind $-CH_2CH_2N(CH_2CH_2OH)_2$, $-CH_2CH_2CH_2N(CH_2CH_2OH)_2$, $-CH_2CH_2N(CH_2CH_2CH_2OH)_2$, $-CH_2CH_2CH_2N(CH_2CH_2CH_2OH)_2$. Ein Beispiel für Arylgruppen ist die Phenylgruppe.

Beispiele für Aryl-($C_1$ bis $C_4$)-alkylgruppen sind die Benzylgruppe und die 2-Phenylethylgruppe.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:

- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1),

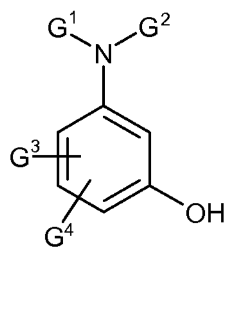

(K1)

worin

$G^1$ und $G^2$ unabhängig voneinander stehen für ein Wasserstoffatom, eine ($C_1$ bis $C_4$)-Alkylgruppe, eine ($C_3$ bis

$C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_2$ bis $C_4$)-Perfluoracylgruppe, eine Aryl-($C_1$ bis $C_6$)-alkylgruppe, eine Amino-($C_1$ bis $C_6$)-alkylgruppe, eine ($C_1$ bis $C_6$)-Dialkylamino-($C_1$ bis $C_6$)-alkylgruppe oder eine ($C_1$ bis $C_6$)-Alkoxy-($C_1$ bis $C_6$)-alkylgruppe, wobei $G^1$ und $G^2$ gemeinsam mit dem Stickstoffatom einen fünfgliedrigen, sechsgliedrigen oder siebengliedrigen Ring bilden können,

$G^3$ und $G^4$ unabhängig voneinander stehen für ein Wasserstoffatom, ein Halogenatom, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$ bis $C_4$)-Polyhydroxyalkylgruppe, eine Hydroxy-($C_1$ bis $C_4$)-alkoxygruppe, eine ($C_1$ bis $C_6$)-Alkyox-($C_2$ bis $C_6$)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2),

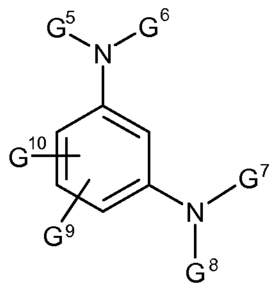

(K2)

worin

$G^5$, $G^6$, $G^7$ und $G^8$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden

$G^9$ und $G^{10}$ unabhängig voneinander stehen für ein Wasserstoffatom, ein Halogenatom, eine ($C_1$ bis $C_4$)-Alkylgruppe, eine ω-(2,4-Diaminophenyl)-($C_1$ bis $C_4$)-alkylgruppe, eine ω-(2,4-Diaminophenyloxy)-($C_1$ bis $C_4$)-alkoxygruppe, eine ($C_1$ bis $C_4$)-Alkoxygruppe, eine Hydroxygruppe, eine ($C_1$ bis $C_4$)-Alkoxy-($C_2$ bis $C_4$)-alkoxygruppe, eine Arylgruppe, eine Heteroarylgruppe, eine ($C_1$ bis $C_4$)-Monohydroxyalkylgruppe, eine ($C_2$ bis $C_4$)-Polyhydroxyalkylgruppe, eine Hydroxy-($C_1$ bis $C_4$)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3),

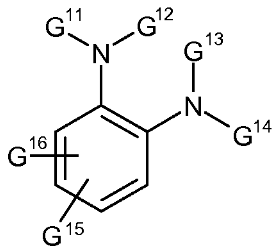

(K3)

worin

$G^{11}$, $G^{12}$, $G^{13}$ und $G^{14}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden

$G^{15}$ und $G^{16}$ unabhängig voneinander stehen für ein Wasserstoffatom, ein Halogenatom, eine Carboxylgruppe, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$ bis $C_4$)-Polyhydroxyalkylgruppe, eine Hydroxy-($C_1$ bis $C_4$)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4),

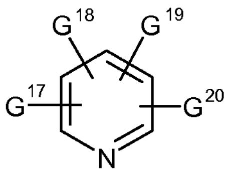

(K4)

worin

$G^{17}$ und $G^{18}$ stehen unabhängig voneinander für eine Hydroxygruppe oder eine Gruppe - $NG^{21}G^{22}$, worin $G^{21}$ und $G^{22}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine Arylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-($C_1$ bis $C_4$)-alkylgruppe,

$G^{19}$ und $G^{20}$ stehen unabhängig voneinander für ein Wasserstoffatom, ein Halogenatom, eine ($C_1$ bis $C_4$)-Alkylgruppe oder eine ($C_1$ bis $C_4$)-Alkoxygruppe.

Es ist bevorzugt, wenn gemäß Formel (K4) die Reste $G^{17}$ und $G^{18}$ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5),

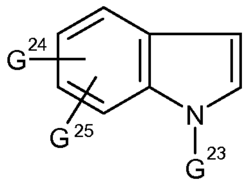

(K5)

worin

- $G^{23}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-($C_1$ bis $C_4$)-alkylgruppe,
- $G^{24}$ steht für eine Hydroxygruppe oder eine Gruppe $-NG^{26}G^{27}$, worin $G^{26}$ und $G^{27}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,
- $G^{25}$ Wasserstoffatom, ein Halogenatom oder eine ($C_1$ bis $C_4$)-Alkylgruppe,

mit der Maßgabe, dass $G^{24}$ in meta-Position oder ortho-Position zum Strukturfragment $NG^{23}$ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6),

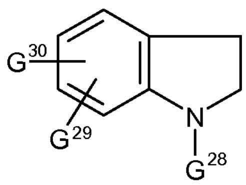

(K6)

worin

- $G^{28}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-($C_1$ bis $C_4$)-alkylgruppe,
- $G^{29}$ steht für eine Hydroxygruppe oder eine Gruppe $-NG^{31}G^{32}$, worin $G^{31}$ und $G^{32}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,
- $G^{30}$ Wasserstoffatom, ein Halogenatom oder eine ($C_1$ bis $C_4$)-Alkylgruppe,

mit der Maßgabe, dass $G^{29}$ in meta-Position oder ortho-Position zum Strukturfragment $NG^{28}$ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für ($C_1$ bis $C_4$)-Alkylreste sind die Gruppen $-CH_3$, - $CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Erfindungsgemäße Beispiele für ($C_3$ bis $C_6$)-Cycloalkylgruppen sind die Cyclopropyl, die Cyclopentyl und die Cyclohexylgruppe. Erfindungsgemäße Beispiele für ($C_1$ bis $C_4$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe. Weiterhin können als bevorzugte Beispiele für eine ($C_1$ bis $C_4$)-Monohydroxyalkylgruppe $-CH_2OH$, $-CH_2CH_2OH$, $-CH_2CH_2CH_2OH$, $-CH_2CH(OH)CH_3$, $-CH_2CH_2CH_2CH_2OH$ genannt werden, wobei die Gruppe $-CH_2CH_2OH$ bevorzugt ist.

Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl- oder Br-Atome, Cl-Atome sind ganz besonders bevorzugte Beispiele. Beispiele für stickstoffhaltige Gruppen sind insbesondere - $NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und - $NH_3^+$. Beispiele für ($C_1$ bis $C_4$)-Monoalkylaminogruppen sind $-NHCH_3$, $-NHCH_2CH_3$, - $NHCH_2CH_2CH_3$, $-NHCH(CH_3)_2$. Beispiele für ($C_1$ bis $C_4$)-Dialkylaminogruppe sind $-N(CH_3)_2$, - $N(CH_2CH_3)_2$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2O-CH_3$, $-CH_2CH_2-O-CH_2CH_3$, $-CH_2CH_2CH_2-O-CH_2CH_3$, $-CH_2CH_2-O-CH(CH_3)_2$, $-CH_2CH_2CH_2-O-CH(CH_3)_2$.

Beispiele für ($C_1$ bis $C_4$)-Alkoxy-($C_1$ bis $C_4$)-alkoxygruppen sind die Gruppen $-O-CH_2CH_2-O-CH_3$, $-O-CH_2CH_2CH_2-O-CH_3$, $-O-CH_2CH_2-O-CH_2CH_3$, $-O-CH_2CH_2CH_2-O-CH_2CH_3$, $-O-CH_2CH_2-O-CH(CH_3)_2$, - $O-CH_2CH_2CH_2-O-CH(CH_3)_2$.

Beispiele für Hydroxy-($C_1$ bis $C_4$)-alkoxyreste sind $-O-CH_2OH$, $-O-CH_2CH_2OH$, $-O-CH_2CH_2CH_2OH$, $-O-CH_2CH(OH)CH_3$, $-O-CH_2CH_2CH_2CH_2OH$

Beispiele für ($C_1$ bis $C_4$)-Aminoalkylreste sind $-CH_2NH_2$, $-CH_2CH_2NH_2$, $-CH_2CH_2CH_2NH_2$, $-CH_2CH(NH_2)CH_3$, $-CH_2CH_2,CH_2CH_2NH_2$. Ein Beispiel für Arylgruppen ist die Phenylgruppe, die auch substituiert sein kann. Beispiele für Aryl-($C_1$ bis $C_4$)-alkylgruppen sind die Benzylgruppe und die 2-Phenylethylgruppe.

Die erfindungsgemäßen Mittel enthalten bevorzugt als farbgebende Verbindung mindestens eine der folgenden Kombinationen a) bis d) von Oxidationsfarbstoffvorprodukten:

a) mindestens einen heterozyklischen Entwickler ausgewählt aus Pyrazolderivaten (insbesondere 1-(2-hydroxyethyl)pyrazol) und Pyrimidinderivaten (insbesondere 2,4,5,6-Tetrahydroxypyrimidon), mindestens eine Verbindung ausgewählt aus m-Aminophenol oder seiner Derivate als Kuppler,
b) 4-Amino-3-methylphenol, 5-Amino-2-methylphenol,
c) p-Toluylendiamin, 4-Amino-3-methylphenol, 5-Amino-2-methylphenol,
d) 2 -(β-Hydroxyethyl)-p-phenylendiamin, 4-Amina-3-methylphenol, 5-Amino-2-methylphenol.

Die erfindungsgemäßen Mittel können zusätzlich mindestens einen direktziehenden Farbstoff enthalten. Dabei

handelt sich um Farbstoffe, die direkt auf das Haar aufziehen und keinen oxidativen Prozess zur Ausbildung der Farbe benötigen. Bevorzugte direktziehende Farbstoffe sind Nitrophenylendiamine, Nitroaminophenole, Azofarbstoffe, Anthrachinone oder Indophenole,

Die direktziehende Farbstoffe werden jeweils bevorzugt in einer Menge von 0,001 bis 20 Ges.-%, bezogen auf die gesamte Anwendungszubereitung, eingesetzt Die Gesamtmenge an direktziehenden Farbstoffen beträgt vorzugsweise höchstens 20 Ges.-%.

Direktziehende Farbstoffe können ferner in anionische, kationische und nichtionische direktziehende Farbstoffe unterteilt werden.

Anionische direktziehende Farbstoffe: Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No, 3; FD&C Yellow No. 6), 2,4-Dinitro-1-naphthol-7-suffansäure-dinatriumsalz (C.I. 10,316; Acid Yellow 1; Food Yellow No, 1), 2-(Indan-1,3-dion-2-yl)chinolin-x,x-sulfonsäure (Gemisch aus Mono- und Disulfionsäure) (C.I. 47,005; D&C Yellow No. 10; Food Yellow No. 13; Acid Yellow 3, Yellow 10), 4-((4-Amino-3-ulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C,I. 19,140; Food Yellow No. 4; Acid Yellow 23), 3-[(4-Phenylamino)phenyl]azobezolsulfonsäuresäure-natriumsalz (C.I, 13,065; Ki406; Acid Yellow 36), 9-(2-Carboxyphenyl)-6-hydroxy-3H-xanthen-3-on (C.I. 45,350; Acid Yellow 73; D&C Yellow No, 8), 5-[(2,4-Dinitrophenyl)amino]-2-phenylaminobenzolsulfonsäure-natriumsalz (C.I. 10,385; Acid Orange 3), 4-[(2,4- Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6- Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5- (Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'-dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2- hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfon-phenyl)azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2- sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau), 3,4,5,6,3',3",5',5"-Octabromphenolsulfonphthalein (Tetrabromphenolblau). Bevorzugte anionische direktziehende Farbstoffe sind die unter den internationalen Bezeichnungen bzw. Handelsnamen Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Kationische direktziehende Farbstoffe: Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1(4H)-naphthalinon-chlorid (C.I. 56,059; Basic Blue No. 99), Bis[4- (dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4- Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4-(dimethylamino)phenyl)-phenylmethanol (C.I. 42,000; Basic Green 4), 1-(2- Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehenden Farbstoffe sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

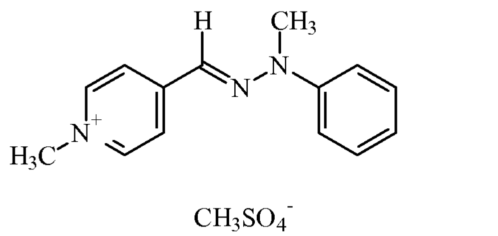

(DZ1)

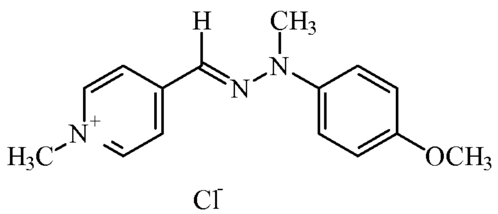

(DZ2)

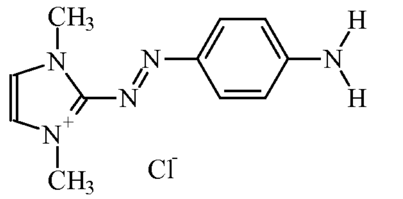

(DZ3)

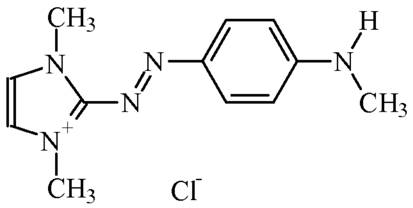

(DZ4)

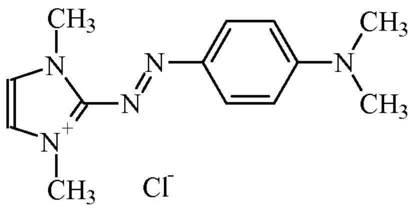

(DZ5)

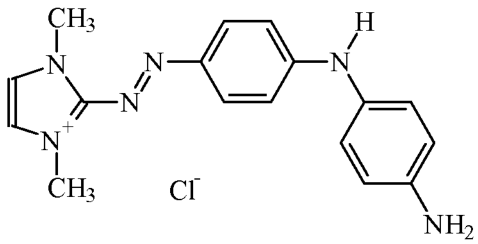

(DZ6)

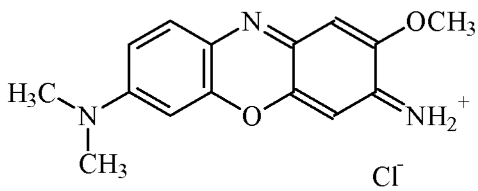

(DZ7)

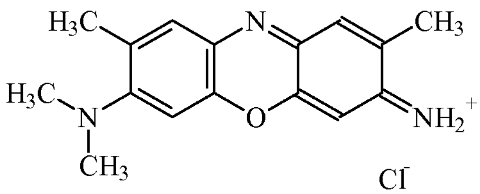

(DZ8)

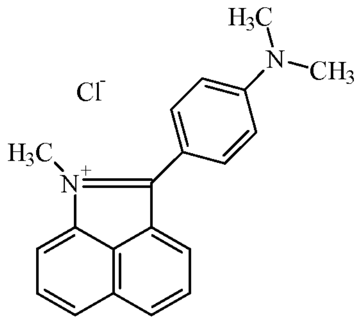

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Nichtionische direktziehende Farbstoffe: Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere: 1,4-Bis[(2-hydroxyethyl)amino]-2-nitrobenzol, 1-(2-Hydroxyethyl)amino-2-nitro-4-[di(2-hydroxyethyl)amino]-benzol (HC Blue 2), 1-Methylamino-4- [methyl-(2,3-dihydroxypropyl)amino]-2-nitrobenzol (HC Blue 6),1-[(2,3- Dihydroxypropyl)-amino]-4-[ethyl-(2-hydroxyethyl)amino]-2-nitrobenzol-hydrochlorid (HC Blue 9), 1-[(2,3-Dihydroxypropyl)amino]-4-[methyl-(2-hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)-amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere: 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere: 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2- Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete Chinonfarbstoffe sind insbesondere: 1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.I. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (C.I. 75,470, Natural Red 4), 1-[(3-Aminopropyl)amino]-4-methylamino-9,10-anthrachinon (HC Blue 8), 1-[(3-Aminopropyl)-amino]-9,10- anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1H- pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere: 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und *-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Als farbgebende Verbindungen der Vorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins der Formel (RN1),

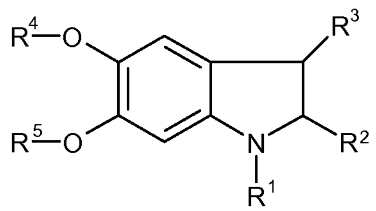

(RN1)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (RN2),

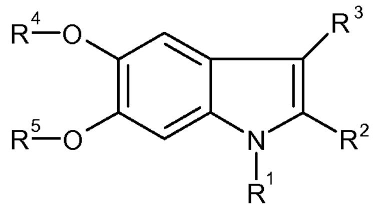

(RN2)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsaure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin-beziehungsweise die Indol-Derivate können sowohl als freie Basen als auch in Form ihrer physiologisch verträglichen Salze mit anorganischen oder organischen Säuren, z. B, der Hydrochloride, der Sulfate und Hydrobromide, eingesetzt werden.

Es ist erfindungsgemäß bevorzugt, wenn das kosmetische Mittel die besagte mittels Propylenoxid modifizierte Stärke Stärke in einer Menge von 0,01 Gew.-% bis 40 Gew.-%, besonders bevorzugt von 0,5 Gew.-% bis 10 Gew.-%, ganz besonders bevorzugt von 2 Gew.-% bis 6 Gew.-%, jeweils bezogen auf das Gewicht des Mittels, enthält.

Die erfindungsgemäßen Mittel enthalten mindestens ein Oxidationsmittel, insbesondere Wasserstoffperoxyd und/oder mindestens ein Anlagerungsprodukt davon. Oxidationsmittel im Sinne der Erfindung sind von Luftsauerstoff verschieden und besitzen ein solches Oxidationspotenzial, das es ermöglicht ein Oxidationsfarbstoffvorprodukt vom Entwicklertyp zu oxidieren Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n $H_2O_2$ (n ist eine positive ganze Zahl größer 0), Harnstoffperoxid und Melaminperoxid, in Frage

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten kosmetischen Färbemittels, in dem kosmetischen Mittel enthalten.

In einer bevorzugten Ausführungsform der Erfindung wird das erfindungsgemäße kosmetische Färbemittel vor

der Applikation aus einer ersten Zusammensetzung, enthaltend in einem kosmetischen Träger mindestens eine farbverändernde Komponente, und einer zweiten Zusammensetzung, enthaltend in einem kosmetischen Träger mindestens ein Oxidationsmittel, gemischt, mit der Massgabe, dass die erste Zusammensetzung oder/und die zweite Zusammensetzung mindestens eine besagte, mittels Propylenoxid modifizierte Stärke enthält. Die erste und zweite Zusammensetzung werden getrennt voneinander in jeweils einem Kompartiment konfektioniert und gemeinsam in einer Verpackungseinheit (Kit) bereitgestellt. Kurz vor der Anwendung werden beide Komponenten miteinander vermischt. Das dabei entstehende gebrauchsfertige Färbepräparat weist bevorzugt einen pH-Wert im Bereich von 6 bis 12, insbesondere von pH 7,5 bis 10, auf.

Das erfindungsgemäße kosmetische Färbemittel enthält bevorzugt mindestens ein Alkalisierungsmittel. Die erfindungsgemäß verwendbaren Alkalisierungsmittel und werden bevorzugt ausgewählt aus mindestens einem Vertreter der Gruppe, die gebildet wird, aus Ammoniak, basischen Aminosäuren, Alkalihydroxiden, Alkanolaminen, Alkalimetallmetasilikaten, Harnstoff, Alkaliphosphaten und Alkalihydrogenphosphaten. Als Alkalimetallionen dienen bevorzugt Lithium, Natrium, Kalium, insbesondere Natrium oder Kalium. Wiederum bevorzugt sind die Alkalisierungsmittel von Ammoniak verschieden.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxyd, L-Arginin, D-Arginin, DL-Arginin, N-Methylglucamin und Harnstoff.

Ferner können die erfindungsgemäßen Färbemittel weitere Wirk-, Hilfs- und Zusatzstoffe, wie beispielsweise nichtionische Polymere; kationische Polymere; zwitterionische und amphotere Polymere; anionische Polymere wie beispielsweise Polyacrylsäuren, vernetzte Polyacrylsäuren, Vinylacetat/Crotonsaure-Copolymere, Vinylpyrrolidon/Vinylacrylat-Copolymere, Vinylacetat/ Butylmaleat/Isobornylacrylat-Copolymere, Methylvinylether/Malein-säureanhydrid-Copolymere und Acrylsäure/Ethylacrylat/N-tertButyl-acrylamid-Terpolymere; haarkonditionierende Verbindungen wie Phospholipide; Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; kationische Tenside; Entschäumer; Antischuppenwirkstoffe ; Lichtschutzmittel, insbesondere derivatisierte Benzophenone, Zimtsäure-Derivate und Triazine; Wirkstoffe wie Allantoin, Pyrrolidoncarbonsäuren und deren Salze sowie Bisabolol; Vitamine, Provitamine und Vitaminvorstufen, insbesondere solche der Gruppen A, $B_3$, $B_5$, $B_6$, C, E, F und H; Pflanzenextrakte insbesondere die Extrakte aus Grünem Tee, Eichenrinde, Brennkessel, Hamamelis, Hopfen, Kamille, Kiettenwurzel, Schachtelhalm, Weißdom, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng oder Ingwerwurzel; Cholesterin; Konsistenzgeber; Fette und Wachse; Komplexbildner; Quell- und Penetrationsstoffe; Trübungsmittel; Perlglanzmittel; festförmige Pigmente; Stabilisierungsmittel für Wasserstoffperoxid und andere Oxidationsmittel; Reibmittel: Antioxidantien; enthalten.

Ein weiterer Gegenstand der vorliegenden Erfindung ist ein kosmetisches Verfahren zur Färbung keratinischer Fasern, bei welchem ein erfindungsgemäßes kosmetisches Mittel auf die keratinischen Fasern (insbesondere menschlichen Haare) aufgetragen wird und nach einer Einwirkungszeit wieder abgespült wird.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt Das Nachwaschen mit einem Shampoo entfällt, wenn ein stark tensidhaltiger Träger, z.B. ein Farbeshampoo, verwendet wurde.

Ein weiterer Gegenstand der vorliegenden Erfindung ist die Verwendung eines erfindungsgemäßen kosmetischen Mittels zur Verbesserung der Farbechtheit künstlicher Färbungen auf keratinischen Fasern, insbesondere menschlichen Haaren.

Die bevorzugten Ausführungsformen des ersten Erfindungsgegenstandes gelten für den zweiten und dritten Erfindungsgegenstand *mutatis mutandis.*

## Beispiele

In die Färbecreme des Handelsprodukts Igora Royal 6-88 (Schwarzkopf) wurden bezogen auf das Gesamtgewicht der Mischung 1,0 Gew.-% einer mit 5,5 Gew.-% Propylenoxid modifizierten Tapiokastärke mit einem mittleren Molekulargewicht $M_w$ von 700 bis 900 kDa, eine Viskosität von 55000 mPas (43 Gew.-% wässrige Lösung) eingearbeitet. Die resultierende modifizierte Färbecreme wurde ummittelbar vor der Applikation auf Haarsträhnen (1 g standardisiertes Haar "European natural hair 6/0" Charge # 06/2010, N93 der Firma Kerling International, Deutschland, an einem Ende zum Haarbündel verklebt) mit einer handelsüblichen 6 Gew.-% Wasserstoffperoxid-haltigen Entwicklerdispersion Oxigenta (Schwarzkopf) im Gewichtsverhältnis 1 zu 1 zu einem erfindungsgemäßen Haarfärbemittel gemischt.

Das anwendungsbereite Färbemittel wurde danach im Gewichtsverhältnis 4 g Färbemittel auf 1 g Haar auf eine Haarsträhne aufgetragen, 30 Minuten bei 32°C einwirken gelassen und von der Faser gespült. Es wurden insgesamt 4 Haarsträhnen damit gefärbt. Darüber hinaus wurden 4 Haarsträhnen nach obiger Vorgehensweise mit dem obigen Handelsprodukt ohne Zusatz der besagten Tapiokastärke (nicht erfindungsgemäß) eingefärbt.

Die Strähnen wurde jeweils trocknen gelassen und farbmetrisch unter Bestimmung der L, a, b Ausgangswerte je Strähne gemessen (Gerät Spectraflash 450, Software Colortools). Je Haarsträhne wurden 8 Messpunkte genommen und für jeden Wert der erfindungsgemäß gefärbten Strähnen in einer Gruppe und der nichterfindungsgemäß gefärbten Strähnen in einer anderen Gruppe das jeweilige arithmetische Mittel der Gruppe unter Erhalt der jeweiligen Werte $L_0$, $a_0$, $b_0$ bestimmt. Genauso wurde bei den folgenden farbmetrischen Messungen verfahren.

Um die Waschechtheit zu ermitteln, wurden die Haarsträhnen einer Waschprozedur unterzogen, die die Haarwäsche simuliert:

> Ein Ultraschallbad wurde mit wässriger Shampoo-Lösung (2 Gew.-% Schaumaschampoo "7 Kräuter") gefüllt. Die colorierten Haarsträhnen wurden in diese Waschlösung getaucht und darin 15 min mit Ultraschall (Stufe 5) behandelt. Diese Behandlung entspricht der Reinigungsleistung von sechs Haarwäschen. Anschließend wurden die Strähnen gründlich gespült, getrocknet und erneut farbmetrisch gemessen. Die Strähnen wurden sodann einem weiteren Waschzyklus unterworfen. Die Bewertung der Waschechtheit wurde über die Berechnung des Farbabstandes der Strähne vor dem Waschen und nach dem jeweiligen Waschzyklus vorgenommen und ermittelt sich aus:

$$\Delta E = [(L_i-L_0)^2 + (a_i - a_0)^2 + (b_i - b_0)^2]^{1/2}$$

$L_i$, $a_i$, $b_i$ Werte nach 6, 12, 18, 24 bzw. 36 Haarwäschen

$L_0$, $a_0$, $b_0$ Werte nach 0 Haarwäschen Haarsträhne

Tabelle 1: ΔE nach 6, 12, 18, 24 und 30 Wäschen

| Anzahl Wäschen | 6 | 12 | 18 | 24 | 30 |
|---|---|---|---|---|---|
| Handelprodukt | 2.4 | 3.6 | 4.5 | 5.7 | 6.8 |
| erfindungsgemäßes Mittel | 2.2 | 3.1 | 3.3 | 3.7 | 4.4 |

## Patentansprüche

1. Kosmetisches Mittel zur Färbung keratinischer Fasern, insbesondere menschlicher Haare, enthaltend in einem kosmetischen Träger mindestens eine farbgebende Verbindung und mindestens eine mittels Propylenoxid modifizierte Stärke, welche ein mittleres Molekulargewicht (Gewichtsmittel) von 700 bis 1000 kDa und einen Propylenoxidgehalt von 2,0 bis 12,0 Gew.-% (bezogen auf das Gewicht der mittels Propylenoxid modifizierten Stärke) besitzt, wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird,
wobei die mittels Propylenoxid modifizierte Stärke eine mittels Propylenoxid modifizierte Tapiokastärke oder eine mittels Propylenoxid modifizierte Kartoffelstärke oder eine mittels Propylenoxid modifizierte Maisstärke oder ein Gemisch aus beiden vorgenannten Stärken, ist, und

wobei zusätzlich mindestens ein Oxidationsmittel, insbesondere Wasserstoffperoxyd und/oder mindestens ein Anlagerungsprodukt davon, enthalten ist.

2. Kosmetisches Mittel nach Anspruch 1, **dadurch gekennzeichnet, dass** die mittels Propylenoxid modifizierte Stärke in einer Menge von 0,01 Gew.-% bis 40 Ges,%, besonders bevorzugt von 0,5 Gew.-% bis 10 Gew,-%, ganz besonders bevorzugt von 2 Gew,-% bis 6 Gew.-%, jeweils bezogen auf das Gewicht des Mittels, enthalten ist.

3. Kosmetisches Mittel nach einem der Ansprüche 1 oder 2, **dadurch gekennzeichnet, dass** die farbgebende Verbindung aus mindestens einer der folgenden Kombinationen a) bis d) von Oxidationsfarbstoffvorprodukten ausgewählt wird:

   a) mindestens einen heterozyklischen Entwickler ausgewählt aus Pyrazolderivaten (insbesondere 1-(2-hydroxyethyl)pyrazol) und Pyrimidinderivaten (insbesondere 2,4,5,6-Tetrahydroxypyrimidon), mindestens eine Verbindung ausgewählt aus m-Aminophenol oder seiner Derivate als Kuppler,
   b) 4-Amino-3-methylphenol, 5-Amino-2-methylphenol,
   c) p-Toluylendiamin, 4-Amino-3-methylphenol, 5-Amino-2-methylphenol,
   d) 2-(β-Hydroxyethyl)-p-phenylendiamin, 4-Amino-3-methylphenol, 5-Amino-2-methylphenol.

4. Kosmetisches Mittel nach einem der Ansprüche 1 bis 3, **dadurch gekennzeichnet, dass** die mittels Propylenoxid modifizierte Stärke in einer 43 Gew.-%-igen Lösung in Wasser eine Viskosität im Bereich von 150 bis 1500000 mPa·s, bevorzugt von 3000 bis 200000 mPa·s, besonders bevorzugt von 10000 bis 100000 mPa·s, ganz besonders bevorzugt von 40000 bis 70000 mPa·s, (Brookfield Viskosimeter, Spindel #7 bei 20°C und 20 U/min) aufweist.

5. Kosmetisches Verfahren zur Färbung keratinischer Fasern, bei welchem ein kosmetisches Mittel nach einem der Ansprüche 1 bis 4 auf die keratinischen Fasern (insbesondere menschlichen Haaren) aufgetragen wird und nach einer Einwirkungszeit wieder abgespült wird.

6. Verwendung eines kosmetischen Mittels nach einem der Ansprüche 1 bis 4, zur Verbesserung der Farbechtheit künstlicher Färbungen auf keratinischen Fasern, insbesondere menschlichen Haaren.

## Claims

1. A cosmetic agent for coloring keratinic fibers, in particular human hair, containing, in a cosmetic carrier, at least one chromophoric compound and at least one propylene oxide-modified starch that has an average molecular weight (weight average) of from 700 to 1,000 kDa and a propylene oxide content of from 2.0 to 12.0 wt.% (based on the weight of the propylene oxide-modified starch),
   wherein the chromophoric compound is selected from at least one oxidation dye precursor of the type of the developer components, and optionally additionally at least one coupler component,
   wherein the propylene oxide-modified starch is a propylene oxide-modified tapioca starch or a propylene oxide-modified potato starch or a propylene oxide-modified maize starch or a mixture of the two aforementioned starches, and
   wherein additionally at least one oxidizing agent, in particular hydrogen peroxide and/or at least one addition product thereof, is contained.

2. The cosmetic agent according to claim 1, **characterized in that** the propylene oxide-modified starch is contained in an amount of from 0.01 wt.% to 40 wt.%, particularly preferably from 0.5 wt.% to 10 wt.%, more particularly preferably from 2 wt.% to 6 wt.%, based in each case on the weight of the agent.

3. The cosmetic agent according to one of claims 1 or 2, **characterized in that** the chromophoric compound is selected from at least one of the following combinations a) to d) of oxidation dye precursors:

   a) at least one heterocyclic developer selected from pyrazole derivatives (in particular 1-(2-hydroxyethyl)pyrazole) and pyrimidine derivates (in particular 2,4,5,6-tetrahydroxypyrimidone), at least one compound selected from m-aminophenol or the derivatives thereof as the coupler,
   b) 4-amino-3-methylphenol, 5-amino-2-methylphenol,
   c) p-toluenediamine, 4-amino-3-methylphenol, 5-amino-2-methylphenol,
   d) 2-(β-hydroxyethyl)-p-phenylene diamine, 4-amino-3-methylphenol, 5-amino-2-methylphenol.

4. The cosmetic agent according to one of claims 1 to 3, **characterized in that** the propylene oxide-modified starch has, in a 43 wt.% solution in water, a viscosity in the range of from 150 to 1,500,000 mPa, preferably from 3,000 to 200,000 mPa, particularly preferably from 10,000 to 100,000 mPa, more particularly preferably from 40,000 to 70,000 mPa (Brookfield viscometer, spindle #7 at 20°C and 20 RPM).

5. A cosmetic method for coloring keratinic fibers, in which a cosmetic agent according to one of claims 1 to 4 is applied to the keratinic fibers (in particular human hair) and rinsed off again after a contact time.

6. The use of a cosmetic agent according to one of claims 1 to 4 for improving the color fastness of synthetic dyes on keratinic fibers, in particular human hair.

## Revendications

1. Agent cosmétique de coloration de fibres de kératine, en particulier de cheveux humains, comprenant dans un support cosmétique au moins un composé de formation de couleur et au moins un amidon, modifié avec de l'oxyde de propylène, qui a un poids moléculaire moyen (moyenne en poids) de 700 à 1000 kDa et une teneur en oxyde de propylène de 2,0 à 12,0 % en poids (sur la base du poids de l'amidon modifié par de l'oxyde de propylène),
le composé de formation de couleur étant choisi parmi au moins un précurseur de colorant d'oxydation du type des composants développeurs et éventuellement en plus au moins un composant copulateur,
l'amidon modifié par de l'oxyde de propylène étant un amidon de tapioca modifié par de l'oxyde de propylène ou un amidon de pomme de terre modifié par de l'oxyde de propylène ou un amidon de maïs modifié par de l'oxyde de propylène ou un mélange de deux amidons susmentionnés, et
au moins un agent d'oxydation, en particulier du peroxyde d'hydrogène, et/ou au moins un produit d'addition de celui-ci, étant contenus.

2. Agent cosmétique selon la revendication 1, **caractérisé en ce que** l'amidon modifié par de l'oxyde de propylène est contenu dans une quantité de 0,01 % à 40 % en poids, de manière particulièrement préférée de 0,5 % à 10 % en poids, de préférence de 2 % à 6 % en poids, à chaque fois sur la base du poids de l'agent.

3. Agent cosmétique selon l'une des revendications 1 ou 2, **caractérisé en ce que** le composé de formation de couleur est choisi parmi au moins une des combinaisons suivantes a) à d) de précurseurs de colorants d'oxydation :

a) au moins un développeur hétérocyclique choisi parmi les dérivés du pyrazole (en particulier le 1-(2-hydroxyéthyl)pyrazole) et les dérivés de la pyrimidine (en particulier la 2,4,5,6-tétrahydroxypyrimidone), au moins un composé choisi parmi le m-aminophénol ou ses dérivés comme copulateur,
b) le 4-amino-3-méthylphénol, le 5-amino-2-méthylphénol,
c) la p-toluylènediamine, le 4-amino-3-méthylphénol, le 5-amino-2-méthylphénol,
d) 2-(β-hydroxyéthyl)-p-phénylènediamine, le 4-amino-3-méthylphénol, le 5-amino-2-méthylphénol.

4. Agent cosmétique selon l'une des revendications 1 à 3, **caractérisé en ce que** l'amidon modifié par de l'oxyde de propylène a dans une solution de 43 % poids dans de l'eau une viscosité de 150 à 1500000 mPa·s, de préférence entre 3000 et 200000 mPa·s, de façon particulièrement préférée de 10000 à 100000 mPa·s, de façon tout particulièrement préférée de 40000 à 70000 mPa·s (viscosimètre Brookfield, broche n°7 à 20 °C et 20 T/min).

5. Procédé cosmétique de coloration de fibres de kératine, dans lequel un agent cosmétique selon l'une des revendications 1 à 4 est appliqué sur les fibres de kératine (en particulier les cheveux humains) et rincé à nouveau après un temps d'action.

6. Utilisation d'un agent cosmétique selon l'une des revendications 1 à 4, pour améliorer la résistance à la décoloration de colorants artificiels sur les fibres de kératine, en particulier les cheveux humains.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1568351 A1
- WO 9801109 A1
- WO 2005082321 A1
- EP 998908 A2